# EUROPÄISCHE PATENTSCHRIFT

(11) **EP 1 603 930 B1**
(45) Veröffentlichungstag und Bekanntmachung des Hinweises auf die Patenterteilung: **29.08.2007**
(21) Anmeldenummer: 04721530.6
(22) Anmeldetag: 18.03.2004
(51) Int. Cl.: C07F 15/00, A61K 31/282, A61P 35/00

(54) **PROTEINBINDENDE DERIVATE VON PLATINKOMPLEXEN MIT CYCLOBUTAN-1,1-DICARBOXYLATLIGANDEN**
PROTEIN-BOUND DERIVATIVES OF PLATINUM COMPLEXES CONTAINING CYCLOBUTANE 1.1-DICARBOXYLLATE LIGANDS
DERIVES SE LIANT A DES PROTEINES ISSUS DE COMPLEXES DE PLATINE COMPRENANT DES LIGANDS DE CYCLOBUTANE-1,1-DICARBOXYLATE

(30) Priorität: 19.03.2003 DE 10314780
(43) Veröffentlichungstag der Anmeldung: 14.12.2005
(73) Patentinhaber: KTB Tumorforschungs GmbH, 79106 Freiburg (DE)
(72) Erfinder: KRATZ, Felix, 79241 Ihringen (DE); WARNECKE, André, 79117 Freiburg (DE)
(74) Vertreter: Dey, Michael
(86) Internationale Anmeldenummer: PCT/EP2004/002850
(87) Internationale Veröffentlichungsnummer: WO 2004/083223

(56) Entgegenhaltungen:
- US-A- 5 011 959
- PASHKOVSKII F.S., KHLEBNIKOVA T.S., LAKHVICH F.A.: "GENERAL APPROACH TO SYNTHESIS OF CARBOPLATIN ANALOG CONTAINING FRAGMENTS OF CARBOXYLIC FATTY ACIDS IN ACID LIGAND" BELARUSKAYA NAVUKA, Bd. 46, Nr. 4, 2002, Seiten 63-65, XP009033021
- BOECKLER C ET AL: "Immunogenicity of new heterobifunctional cross-linking reagents used in the conjugation of synthetic peptides to liposomes" JOURNAL OF IMMUNOLOGICAL METHODS, ELSEVIER SCIENCE PUBLISHERS B.V.,AMSTERDAM, NL, Bd. 191, Nr. 1, 10. Mai 1996 (1996-05-10), Seiten 1-10, XP004020848 ISSN: 0022-1759
- LEBWOHL D ET AL: "Clinical development of platinum complexes in cancer therapy: an historical perspective and an update" EUROPEAN JOURNAL OF CANCER, PERGAMON PRESS, OXFORD, GB, Bd. 34, Nr. 10, September 1998 (1998-09), Seiten 1522-1534, XP004285042 ISSN: 0959-8049

## Beschreibung

Die Erfindung betrifft niedermolekulare Platinkomplexe von Cyclobutan-1,1-dicarboxylatliganden, die eine proteinbindende Gruppe enthalten, deren Herstellung und Verwendung.

Carboplatin (Diamminplatin(II)-cyclobutan-1,1-dicarboxylat) ist ein antineoplastisch wirksamer Platin(II)-Komplex, der zur Behandlung verschiedener Krebserkrankungen eingesetzt wird (Woloschuk, D. M. et al., Drug lntell. Clin. Pharm. 1988, 22, 843-849). Von dem strukturell verwandten Cisplatin (*cis*-Diammindichloroplatin(II)) unterscheidet sich Carboplatin durch Austausch der Chloroliganden durch einen chelatisierenden Cyclobutan-1,1-dicarboxylatliganden, woraus eine veränderte Pharmakokinetik sowie ein von Cisplatin abweichendes Toxizitätsprofil resultiert (Lokich, J., Cancer Invest. 2001, 19, 756-760). Jedoch sind Therapien mit Carboplatin von Nebenwirkungen begleitet (Go, R. S. et al., J Clin. Oncol. 1999, 17, 409-422). Es wurde bereits vorgeschlagen, zur Verbesserung des Nebenwirkungsprofils und der Wirksamkeit von Zytostatika proteinbindende Formulierungen zu entwickeln, welche *in vivo* an endogene Serumproteine koppeln und auf diese Weise makromolekulare Transportformen der Wirkstoffe darstellen (Kratz, F. et al., Am. Assoc. Cancer Res. 2001, 42, 138-139, Kratz, F. et al., J. Med. Chem. 2000, 43, 1253-1256).

Der Erfindung liegt die Aufgabe zugrunde, proteinbindende Derivate von Carboplatin zu schaffen, welche weniger unerwünschte Nebenwirkungen und höhere Wirksamkeit gegenüber Tumorgewebe aufweisen.

Diese Aufgabe wird erfindungsgemäß gelöst durch niedermolekulare Carboplatin-Derivate der allgemeinen Formel I 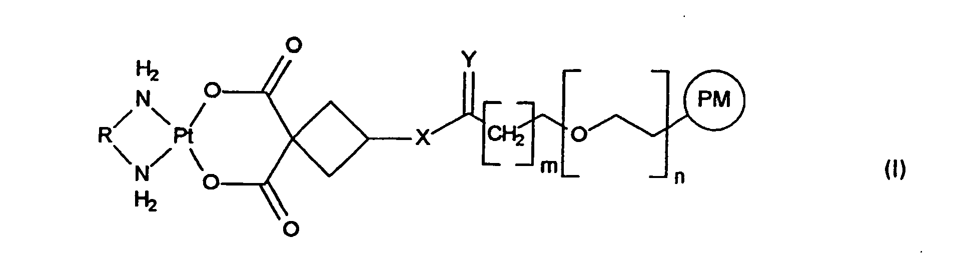 worin R = 2 H, 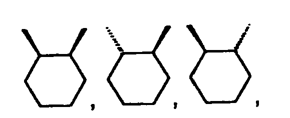 -(CH₂)ᵢ- (i = 2 oder 3), X = O oder NH, Y = O, S oder 2 H, n = 0 bis 5, m = 0 bis 6 bedeuten und PM eine Proteinbindende Gruppe ist.

Die erfindungsgemäßen Verbindungen sind aus einem antitumoral wirksamen cis-konfigurierten Platin(II)-Komplex und einem heterobifunktionellen Crosslinker aufgebaut. Im Folgenden wird dieser Aufbau näher erläutert:

Der antitumoral wirksame Platinkomplex ist ein Wirkstoffderivat der allgemeinen Formel II 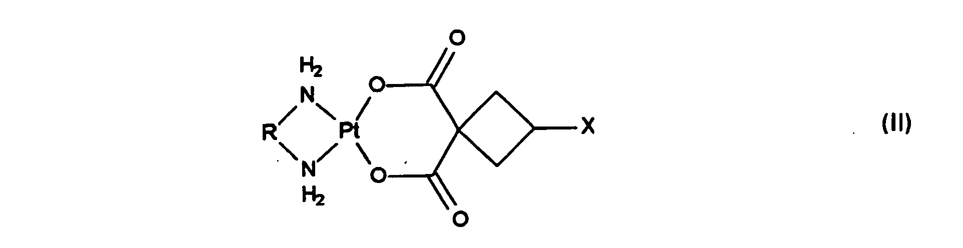 worin
R = 2 H, 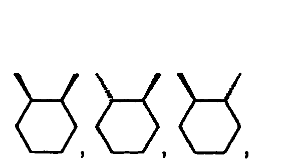 -(CH₂)ᵢ- (i = 2 oder 3)
X = OH oder NH₂
bedeuten. Von dem klinischen Standard Carboplatin unterscheidet sich dieser durch das Vorliegen einer Hydroxy- oder Aminogruppe am Cyclobutanring sowie gegebenenfalls durch Vorliegen chelatisierender Aminliganden wie *trans*-1,2-Diaminocyclohexan, *cis*-1 ,2-Diaminocyclohexan, Ethylendiamin oder 1,3-Diaminopropan.

Der heterobifunktionelle Crosslinker ist ein Carbonsäure-Derivat oder ein Alkohol mit einer proteinbindenden Gruppe der allgemeinen Formel III 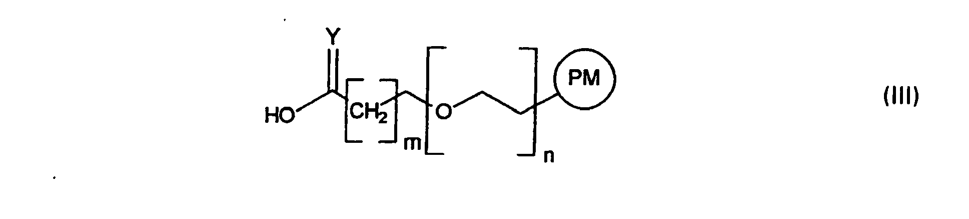 in der
Y = 2 H, O oder S
n = 0 bis 5
m = 0 bis 6
PM = Proteinbindende Gruppe
bedeuten.

Die proteinbindende Gruppe (PM) ist bevorzugt ausgewählt unter einer 2-Dithiopyridylgruppe, einer Halogenacetamidgruppe, einer Halogenacetatgruppe, einer Disulfidgruppe, einer Acrylsäureestergruppe, einer Monoalkylmaleinsäureestergruppe, einer Monoalkylmaleaminsäureamidgruppe, einer *N*-Hydroxysuccinimidylestergruppe, einer Isothiocyanatgruppe, einer Aziridingruppe oder einer Maleinimidgruppe. Eine besonders bevorzugte proteinbindende Gruppe ist die Maleinimidgruppe.

Erfindungsgemäße Verbindungen werden formal durch Kondensation des Wirkstoffderivats mit dem Crosslinker erhalten. Zwischen Wirkstoffderivat und Crosslinker liegt daher eine Esterbindung, eine Amidbindung, eine Thioesterbindung, eine Thioamidbindung, eine Etherbindung oder eine Aminbindung vor.

Die Herstellung der erfindungsgemäßen Platinkomplexe erfolgt bevorzugt durch Umsetzung eines Cyclobutan-1,1-dicarbonsäure-Derivats der allgemeinen Formel IV 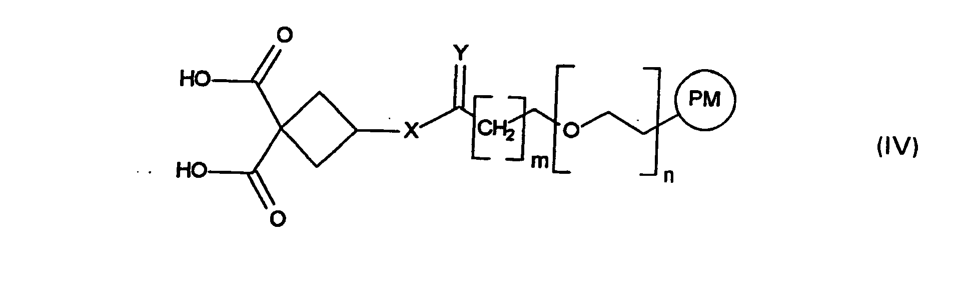 in der
X = O oder NH
Y = O, S oder 2 H
m = 0 bis 5
n = 0 bis 6
und PM eine Proteinbindende Gruppe bedeuten, mit einem Platinkomplex der allgemeinen Formel V 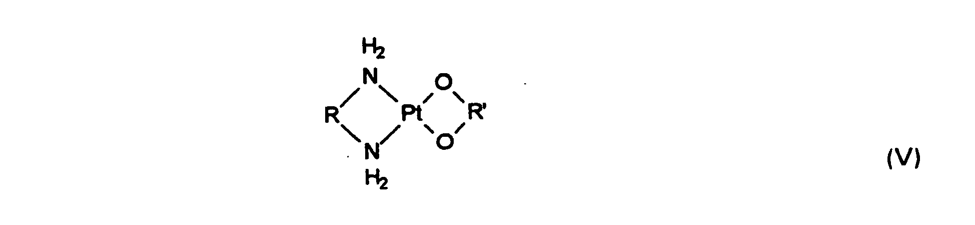 in der
R = 2 H, 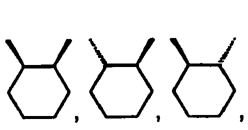 -(CH₂)ᵢ- (i = 2 oder 3)
R' = 2 NO₂, SO₂ oder CO bedeuten.

Zur Umsetzung kann z.B. der Platinkomplex in Wasser gelöst und mit dem Cyclobutan-1,1-dicarbonsäure-Derivat oder einem Alkali- oder Erdalkalimetallsalz des Cyclobutan-1,1-dicarbonsäure-Derivats versetzt werden. Die Umsetzungen werden zweckmäßig bei Temperaturen zwischen 0 °C und 50 °C durchgeführt, wobei die Reaktionszeit normalerweise zwischen 1 und 24 Stunden liegt. Die Produktisolierung kann durch übliche Methoden wie Kristallisation, Chromatographie an Kieselgel oder Reversed-Phase-Chromatographie (präparative HPLC) erfolgen.

Nach einer bevorzugten Ausführungsweise wird eine wässrige Lösung eines *cis*-Diaminoalkylplatin(II)dinitrat-Komplexes oder cis-Diamminplatin(II)dinitratmiteinemCyclobutan-1,1-dicarbonsäure-Derivat, welches eine Maleinimidgruppe und ein Oligo(ethylenglykol)-Rückgrat aufweist, bei einem pH-Wert zwischen 5 und 6 umgesetzt. Die Reinigung der so erhaltenen Platin komplexe erfolgt vorzugsweise durch Säulenchromatographie an Kieselgel oder durch Reversed-Phase-Chromatographie (siehe Beispiele 5, 6 und 9). Auf diese Weise erhaltene Platinkomplexe weisen eine exzellente Wasserlöslichkeit auf.

Die für die Komplexbildung bevorzugt eingesetzten Cyclobutan-1,1-dicarbonsäure-Derivate mit einer Maleinimidgruppe und einem Oligo(ethylenglykol)-Rückgrat lassen sich in einer vierstufigen Synthese ausgehend von Bis(4-methoxybenzyl)malonat und 1,3-Dibrom-2-*tert*.-butyldimethylsiloxypropan herstellen:

Im ersten Schritt erfolgt eine Dialkylierung von Bis(4-methoxybenzyl)malonat mit 1,3-Dibrom-2-tert.-butylsiloxypropan, die zum Cyclobutanringsystem führt.

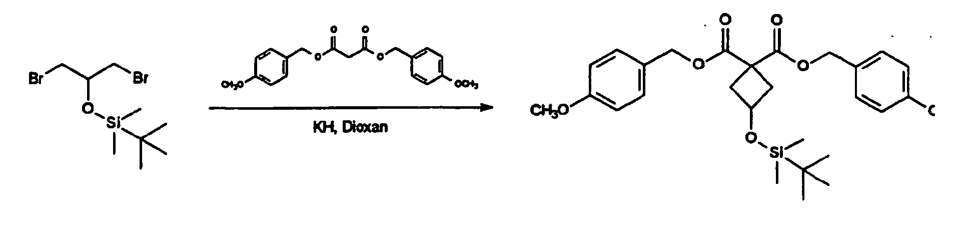

Diese Reaktion wird bevorzugt in einem aprotisch polaren Lösungsmittel wie Dioxan oder DMF unter Benutzung von Basen wie Kaliumhydrid, Natriumhydrid oder Natriumhexamethyldisilazid bei Temperaturen > 100 °C ausgeführt. Typischerweise beträgt die Reaktionszeit unter diesen Bedingungen 48-120 Stunden (siehe Beispiel 1).

Im zweiten Schritt erfolgt die Abspaltung der tert.-Butyldimethylsilyl-Schutzgruppe nach Methoden, die dem Fachmann geläufig sind, bevorzugt unter Einsatz von Tetrabutylammoniumfluorid in Tetrahydrofuran (siehe Beispiel 2).

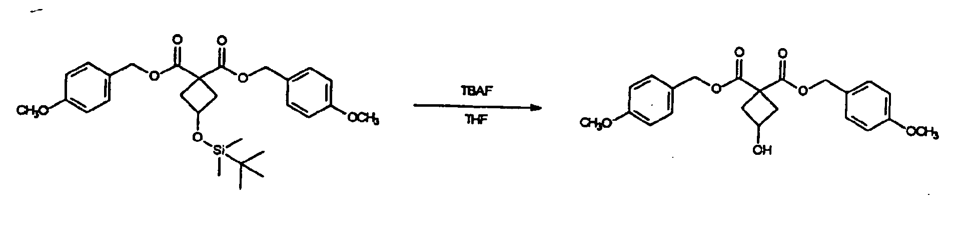

Im dritten Schritt erfolgt die Veresterung der 3-Hydroxygruppe des Cyclobutanrings von Bis(4-methoxybenzyl)-3-hydroxycyclobutan-1,1-dicarboxylat mit einer Maleinimidocarbonsäure der allgemeinen Formel VIa. 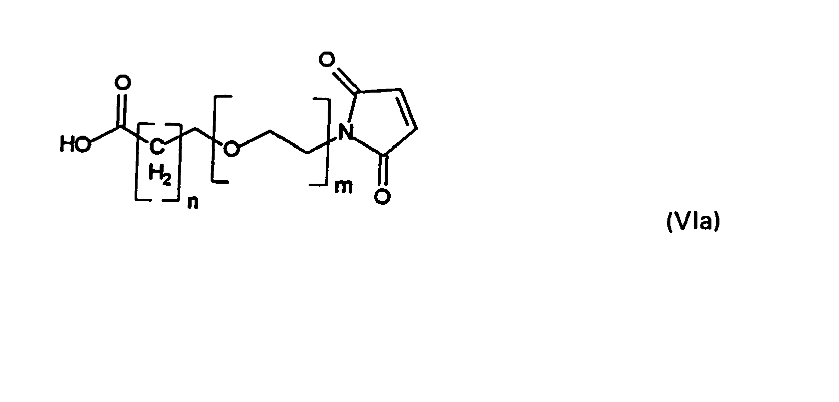 in der
n = 0 bis 5
m = 0 bis 6
bedeuten.

Dabei werden als Reagenzien zur Aktivierung der Carboxylgruppe des Crosslinkers vorzugsweise *N,N'*-Dicyclohexylcarbodiimid, *N,N*'-Diisopropylcarbodiimid, (Benzotriazol-1-yloxy)tris(dimethylamino)phosphonium-Hexafluorophosphat oder 2-Chlor-1-methylpyridiniumiodid unter Zusatz gängiger Katalysatoren bzw. Hilfsbasen wie z.B. Trialkylamine, Pyridin, 4-Dimethylaminopyridin (DMAP) oder Hydroxybenzotriazol (HOBt) eingesetzt. Die Umsetzung erfolgt zweckmäßig in einem polaren organischen Lösungsmittel, vorzugsweise in Dichlormethan, *N,N*-Dimethylformamid und Tetrahydrofuran. Die Umsetzungen werden zweckmäßig bei Temperaturen zwischen -10 °C bis Raumtemperatur durchgeführt, wobei die Reaktionszeit normalerweise zwischen 3 und 48 Stunden liegt.

Nach einer bevorzugten Ausführungsweise wird Bis(4-methoxybenzyl)-3-hydroxycyclobutan-1,1-dicarboxylatmiteinerMaleinimidocarbonsäure, welche ein Oligo(ethylenglykol)-Rückgrataufweist, unter Einsatz von 2-Chlor-1-methylpyridiniumiodid umgesetzt (siehe Beispiele 3 und 7) unter Bildung einer Verbindung der allgemeinen Formel VII.

Im vierten Schritt erfolgt die Abspaltung der 4-Methoxybenzyl-Schutzgruppen mit Trifluoressigsäure und Anisol bei 0°C (siehe Beispiele 4 und 8).

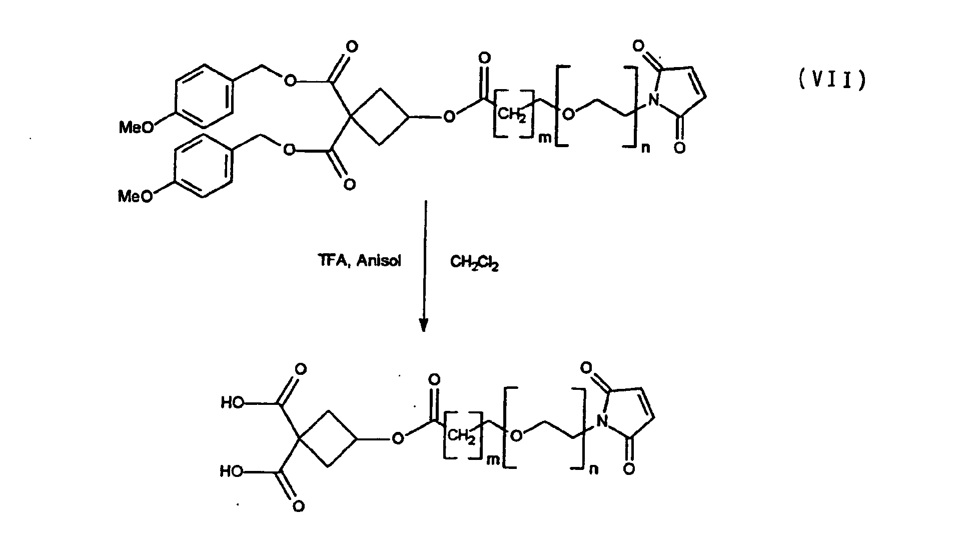

Ein wesentliches Merkmal der erfindungsgemäßen Platinkomplexe liegt in einer raschen kovalenten Bindung an Serumproteine über die proteinbindende Gruppe, wodurch eine makromolekulare Transportform des Wirkstoffs generiert wird. Von Serumproteinen wie Transferrin, Albumin und LDL ist eine erhöhte Aufnahme in Tumorgewebe bekannt (Kratz F.; Beyer U. Drug Delivery 1998, 5, 281-299), sodass diese im Rahmen der Erfindung als endogene Träger für Zytostatika herangezogen werden können. Ein besonders bevorzugtes Serumprotein ist zirkulierendes Humanserumalbumin (HSA), das mit einer durchschnittlichen Konzentration von 30 bis 50 g/L die Hauptprotein-Komponente des menschlichen Blutes bildet (Peters T. Adv. Protein Chem. 1985, 37, 161-245) und eine freie Cysteingruppe (Cystein-34-Gruppe) an der Oberfläche des Proteins aufweist, welche zur Anbindung von thiolbindenden Gruppen wie Maleinimiden oder Disulfiden geeignet ist (WO 00/76551).

Die erfindungsgemäßen proteinbindenden Platinkomplexe können als Arzneimittel parenteral, bevorzugt intravenös appliziert werden. Dazu werden die erfindungsgemäßen Platinkomplexe als Lösungen, Feststoffe oder Lyophilisate, gegebenenfalls unter Verwendung üblicher Hilfsstoffe bereitgestellt. Solche Hilfsstoffe sind beispielsweise Polysorbate, Glucose, Lactose, Mannitol, Dextrane, Zitronensäure, Tromethamol, Triethanolamin, Aminoessigsäure und/oder synthetische Polymere. Die applizierten Komplexe reagieren dann mit Serumproteinen unter Bildung der Transportform.

Die Reaktion der neuen Platinkomplexe mit Serumproteinen kann alternativ auch extrakorporal durchgeführt werden, z.B. mit einer zur Infusion vorgesehenen Albumin-, Blut- oder Serummenge.

Protein-gebundene Platinkomplexe gemäß der Erfindung weisen gegenüber herkömmlichen niedermolekularen Komplexen eine veränderte Pharmakokinetik auf und reichern sich aufgrund ihres makromolekularen Charakters in Tumorgewebe an. Durch eine intrazellulär stattfindende Hydrolyse-Reaktion wird der labil-gebundene Cyclobutan-1,1-dicarboxylatligand abgespalten und dabei werden Aquo-, Hydroxy- oder gemischte Aquohydroxykomplexe als aktive Komponenten freigesetzt. In Tierversuchsstudien zeigten diese proteinbindenden Platinkomplexe eine höhere Wirksamkeit als der klinische Standard Carboplatin (siehe Beispiel 10):

Die folgenden Beispiele erläutern die Erfindung in Verbindung mit der Zeichnung näher. Die Zeichnung stellt graphisch die Ergebnisse eines Tierversuchs mit einer Substanz gemäß Erfindung im Vergleich zu Carboplatin anhand der Änderung des Tumorvolumens dar.

### Beispiel 1

### Herstellung von Bis(4-methoxybenzyl)-3-tert.-butyldimethyl-siloxycyclobutan-1,1-dicarboxylat (PMB-CB-OTBS)

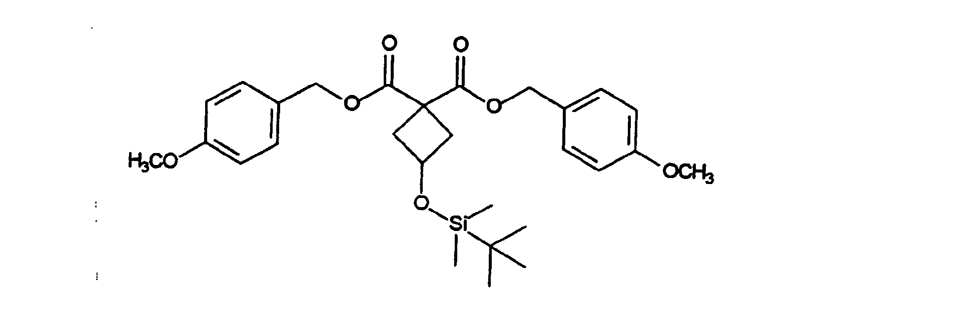

Zu einer Suspension von Kaliumhydrid (35 % in Mineralöl) (2.10 g, 18.29 mmol) in 20 mL wasserfreiem Dioxan wird unter Inertgasatmosphäre Bis(4-methoxybenzyl)malonat (6.00 g, 17.4 mmol) innerhalb 30 min zugetropft. Man lässt weitere 1 5 min bei Raumtemperatur rühren, versetzt mit 1,3-Dibrom-2-*tert*.-butyldimethylsiloxypropan (6.08 g, 18.29 mmol) und erhitzt über Nacht unter Rückfluss. Zu der auf Raumtemperatur abgekühlten Reaktionsmischung wird Kaliumhydrid (35 % in Mineralöl) (2.10 g, 18.29 mmol) als Suspension in 10 mL Dioxan langsam gegeben. Anschließend wird drei weitere Tage unter Rückfluss gerührt. Das gebildete Kaliumbromid wird über Celite abfiltriert, das Lösungsmittel im Vakuum entfernt und der Rückstand säulenchromatographisch (Kieselgel, Ethylacetat/Isohexan 1:10) gereinigt. Man erhält 2.78 g PMB-CB-OTBS (31 % d. Theorie) als farbloses Öl.

### Beispiel 2

### Herstellung von Bis(4-methoxybenzyl)-3-hydroxycyclobutan-1,1-di-carboxylat (PMB-CB-OH)

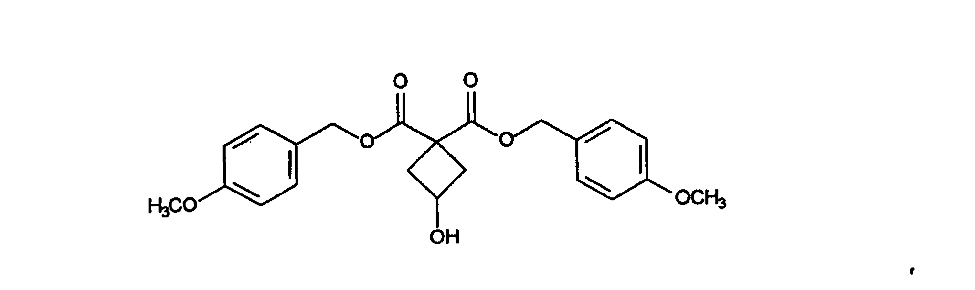

Eine Lösung von PMB-CB-OTBS (2.69 g, 5.23 mmol) in 25 mL wasserfreiem THF wird mit Tetrabutylammoniumfluorid (2.47 g, 7.85 mmol) versetzt und 15 min bei Raumtemperatur gerührt. Anschließend wird das Lösungsmittel im Vakuum entfernt und der Rückstand säulenchromatographisch (Kieselgel, Ethylacetat/Isohexan 1:1) gereinigt. Man erhält 1.85 g PMB-CB-OH (88 % d. Theorie) als farbloses Öl, das langsam kristallisiert.

### Beispiel 3

### Herstellung von Bis(4-methoxybenzyl)-3-(6-maleinimido-4-oxacaproyl)cyclobutan-1,1-dicarboxylat (PMB-CB-1-Mal)

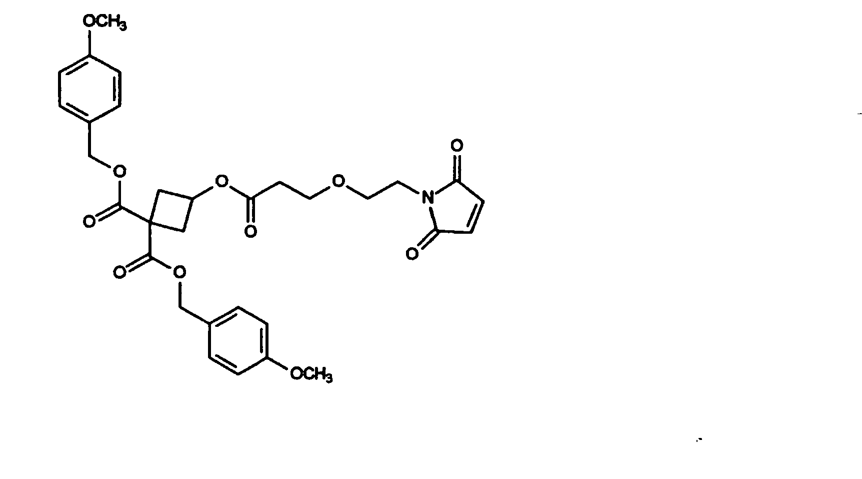

Eine Lösung von PMB-CB-OH (1.97 g, 4.91 mmol), 6-Maleinimido-4-oxacapronsäure (1.57 g, 7.37 mmol) und Triethylamin (2.04 mL, 8.64 mmol) in 30 mL wasserfreiem Dichlormethan wird mit 2-Chlor-1-methylpyridiniumiodid (1.88 g, 7.37 mmol) versetzt und drei Stunden bei Raumtemperatur gerührt. Man verdünnt mit 140 mL Dichlormethan, wäscht jeweils zweimal mit 80 mL Salzsäure 1 n, 80 mL Wasser sowie einmal mit 80 mL Natriumchloridlösung (ges.) und trocknet über Magnesiumsulfat. Anschließend wird das Lösungsmittel im Vakuum entfernt und der Rückstand säulenchromatographisch (Kieselgel, Chloroform/Methanol 200:1) gereinigt. Man erhält 2.89 g PMB-CB-1-Mal (99 % d. Theorie) als farbloses Öl.

### Beispiel 4

### Herstellung von 3-(6-Maleinimido-4-oxacaproyl)cyclobutan-1,1-di-carbonsäure (COOH-CB-1-Mal)

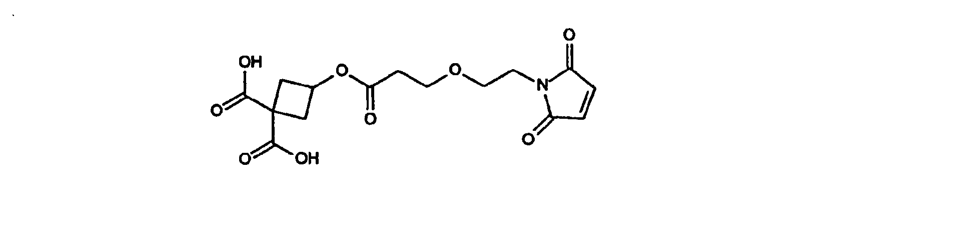

Eine Lösung von PMB-CB-1-Mal (2.76 g, 4.63 mmol) und Anisol (7.57 mL, 69.5 mmol) in 50 mL wasserfreiem Dichlormethan wird bei 0 °C mit 10 mL Trifluoressigsäure versetzt und zwei Stunden bei 0 °C gerührt. Anschließend wird das Lösungsmittel im Vakuum entfernt und der Rückstand säulenchromatographisch (Kieselgel, Chloroform/Methanol 20:1) gereinigt. Man erhält 1.28 g COOH-CB-1-Mal (78 % d. Theorie) als farblosen Sirup.

### Beispiel 5

### Herstellung von trans-(R,R/S,S)-Cyclohexan-1,2-diaminoplatin(II)-[3-(6-maleinimido-4-oxacaproyl)cyclobutan-1,1-dicarboxylat] (DACH-Pt-CB-1-Mal)

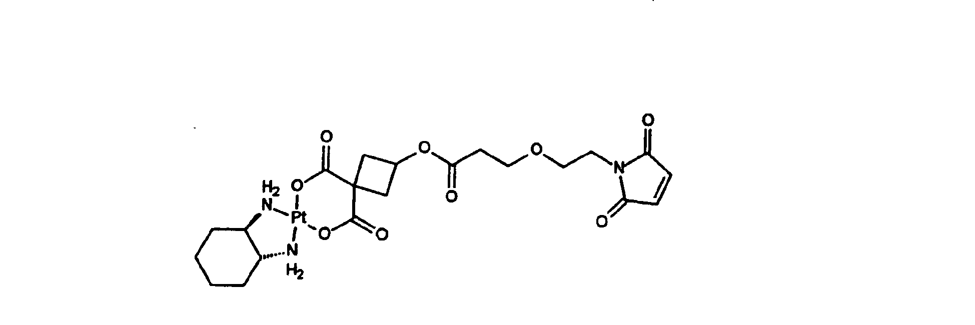

Zu einer auf Raumtemperatur abgekühlten wässrigen Lösung von [Pt *trans*-DACH](NO₃)₂ (300 mg, 692 *µ*mol), die man durch Erhitzen (50 °C) des Platinkomplexes mit 60 mL Wasser erhält, wird eine Lösung von COOH-CB-1-Mal (271 mg, 762 *µ*mol) in 5 mL Wasser gegeben. Die Reaktionsmischung wird mit 0.1 M KOH auf pH 5.5 eingestellt und drei Stunden bei Raumtemperatur in der Dunkelheit gerührt. Anschließend wird das Lösungsmittel im Vakuum entfernt und der Rückstand durch Säulenchromatographie (Kieselgel, Ethanol) gereinigt. Man erhält 291 mg DACH-Pt-CB-1-Mal (61 % d. Theorie) als farblosen Feststoff.
¹H-NMR (CD₃OD):
δ = 0.97-1.29 (m, 4H, Cyclohexyl-H), 1.38-1.56 (m, 2H, Cyclohexyl-H), 1.81-1.96 (m, 2H, Cyclohexyl-H), 2.19-2.36 (m, 2H, Cyclohexyl-H), 2.42 (t, J = 5.9 Hz, CH₂COO), 2.60-2.79 (m, 2H, CH₂CHOR), 3.27-3.41 (m, 2H, CH₂CHOR), 3.46-3.66 (m, 6H, NCH₂, OCH₂), 4.67-4.86 (m, 1 H, CHOR), 6.76 (s, 2H, C(O)CH = CHCO)
¹³C-NMR (CD₃OD):
δ = 25.51, 33.30 (Cyclohexyl), 35.92 (CH₂COO), 38.17 (NCH₂), 39.80/40.09 (CH₂CHOR), 51.35 (C(COOH)₂), 63.81/63.86 (Cyclohexyl-NH₂), 65.66 (CHOR), 67.22, 68.65 (OCH₂), 135.50 (C(O)CH = CHCO), 172.52, 172.80 (C(O)CH = CHCO, CH₂COO), 180.41, 180.61 (C(COOH)₂)
ESI-MS (4.0 kV, MeOH):
m/z (%) 663.0 ([M+1]⁺, 100)

### Beispiel 6

### Herstellung von Diamminplatin(11)-[3-(6-maleinimido-4-oxacaproyl) cyclobutan-1,1-dicarboxylat] ((NH₃)₂-Pt-CB-1-Mal)

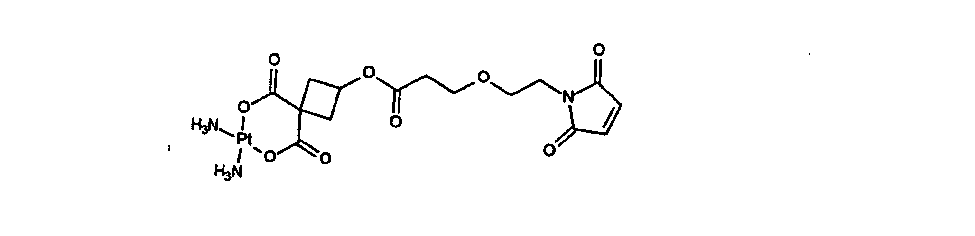

Zu einer auf Raumtemperatur abgekühlten wässrigen Lösung von [(NH₃)₂Pt](NO₃)₂ (485 mg, 1.37 mmol), die man durch Erhitzen (50 °C) des Platinkomplexes mit 90 mL Wasser erhält, wird eine Lösung von COOH-CB-1-Mal (538 mg, 1.51 mmol) in 5 mL Wasser gegeben. Die Reaktionsmischung wird mit 0.1 M KOH auf pH 5.5 eingestellt und drei Stunden bei Raumtemperatur in der Dunkelheit gerührt. Anschließend wird das Lösungsmittel im Vakuum entfernt und der Rückstand durch Säulenchromatographie (Kieselgel, Ethanol) gereinigt. Man erhält 263 mg (NH₃)₂-Pt-CB-1-Mal (33 % d. Theorie) als farblosen Feststoff.
ESI-MS:
m/z (%) 581.8 ([M]⁺, 100)

### Beispiel 7

### Herstellung von Bis(4-methoxybenzyl)-3-(15-maleinimido-4,7,10, 13-tetroxapentadecanoyl)cyclobutan-1,1-dicarboxylat (PMB-CB-4-Mal)

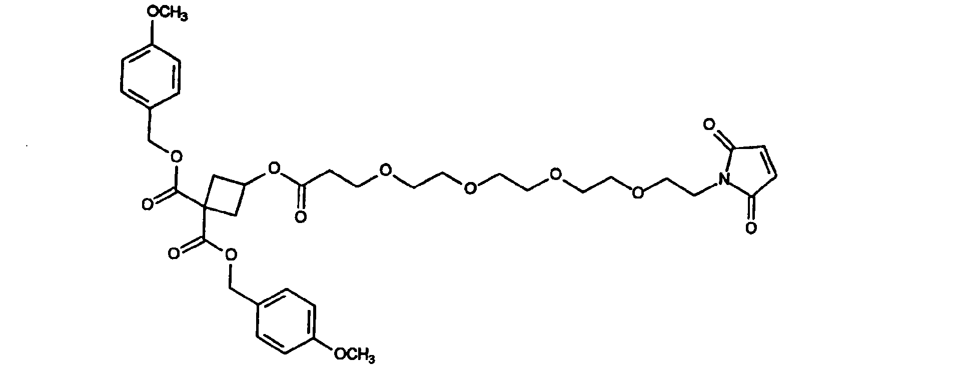

Eine Lösung von PMB-CB-OH (1.154 g, 2.881 mmol), 15-Maleinimido-4,7,10,13-tetroxapentadecansäure (1.49 g, 4.32 mmol) und Triethylamin (1.20 mL, 8.64 mmol) in 20 mL wasserfreiem Dichlormethan wird mit 2-Chlor-1-methylpyridiniumiodid (1.10 g, 4.32 mmol) versetzt und drei Stunden bei Raumtemperatur gerührt. Man verdünnt mit 80 mL Dichlormethan, wäscht jeweils zweimal mit 50 mL Salzsäure 1 n, 50 mL Wasser sowie einmal mit 50 mL Natriumchloridlösung (ges.) und trocknet über Magnesiumsulfat. Anschließend wird das Lösungsmittel im Vakuum entfernt und der Rückstand säulenchromatographisch (Kieselgel, Ethylacetat) gereinigt. Man erhält 1.93 g PMB-CB-4-Mal (92 % d. Theorie) als farbloses Öl.

### Beispiel 8

### Herstellung von 3-(15-Maleinimido-4,7,10,13-tetroxapentadecanoyl)cyclobutan-1,1-dicarbonsäure (COOH-CB-4-Mal)

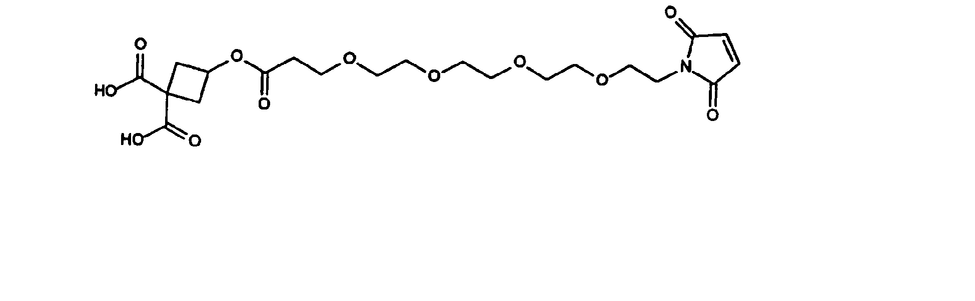

Eine Lösung von PMB-CB-4-Mal (1.94 g, 2.66 mmol) und Anisol (4.35 mL, 39.9 mmol) in 50 mL wasserfreiem Dichlormethan wird bei 0 °C mit 10 mL Trifluoressigsäure versetzt und zwei Stunden bei 0 °C gerührt. Anschließend wird das Lösungsmittel im Vakuum entfernt und der Rückstand säulenchromatographisch (Kieselgel, Chloroform/Methanol 10: 1) gereinigt. Man erhält 1.15 g COOH-CB-4-Mal (89 % d. Theorie) als farblosen Sirup.

### Beispiel 9

### Herstellung von trans-(RR/SS)-Cyclohexan-1,2-diaminoplatin(II)-[3-(15-maleinimido-4,7,10,13-tetroxapentadecanoyl)cyclo-butan-1,1-dicarboxylat]

### (DACH-Pt-CB-4-Mal)

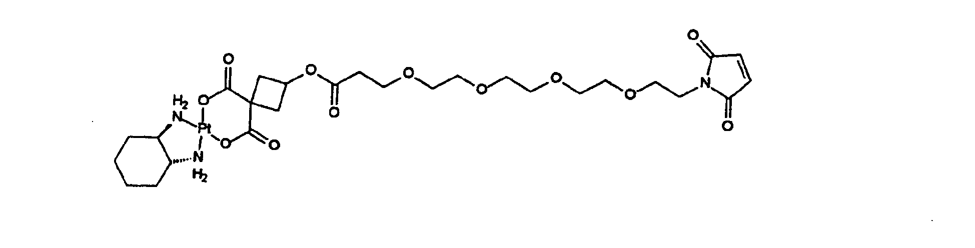

Zu einer auf Raumtemperatur abgekühlten wässrigen Lösung von [Pt *trans*-DACH](NO₃)₂ (175 mg, 405 *µ*mol), die man durch Erhitzen (50 °C) des Platinkomplexes mit 30 mL Wasser erhält, wird eine Lösung von COOH-CB-4-Mal (217 mg, 445 *µ*mol) in 5 mL Wasser gegeben. Die Reaktionsmischung wird mit 0.1 M KOH auf pH 5.5 eingestellt und drei Stunden bei Raumtemperatur in der Dunkelheit gerührt. Anschließend engt man im Vakuum auf ca. 5 mL ein und zentrifugiert den Niederschlag ab. Der Überstand wird durch präparative HPLC (Nucleosil^{®} 100-7-C18-Säule (250 x 15 mm), Wasser/Acetonitril 80:20 + 0.05 % TFA, Fluss: 20 mL/min, Retentionszeit: ca. 20 min) gereinigt. Nach Entfernen des Laufmittels im Vakuum und Kristallisation des Rückstandes durch Zugabe von ca. 1 mL 2-Propanol erhält man 60 mg DACH-Pt-CB-4-Mal (19 % d. Theorie) als farblosen Feststoff.
¹H-NMR (D₂O, geeicht auf Aceton δ ≡ 2.20 ppm):
δ = 0.98-1.35 (m, 4H, Cyclohexyl-H), 1.44-1.62 (m, 2H, Cyclohexyl-H), 1.94-2.06 (m, 2H, Cyclohexyl-H), 2.28-2.48 (m, 2H, Cyclohexyl-H), 2.66 (t, J = 6.0 Hz, CH₂COO), 2.80-2.93 (m, 2H, CH₂CHOR), 3.34-3.50 (m, 2H, CH₂CHOR), 3.54-3.84 (m, 18H, NCH₂, OCH₂), 4.93 ('p', J = 7.1 Hz, 1 H, CHOR), 6.85 (s, 2H, C(O)CH = CHCO)
¹³C-NMR (D₂O, geeicht auf Aceton):
δ = 24.30, 32.22 (Cyclohexyl), 34.91 (CH₂COO), 37.37 (NCH₂), 38.54/38.72 (CH₂CHOR), 50.71 (C(COOH)₂), 63.05/63.09 (Cyclohexyl-NH₂), 65.37 (CHOR), 66.52, 68.02, 69.73, 69.97, 70.01 (OCH₂), 134.86 (C(O)CH=CHCO), 173.39, 174.11 (C(O)CH=CHCO, CH₂CCOO), 180.30, 180.47 (C(COOH)₂)
¹⁹⁵Pt-NMR (D₂O): δ = -311
IR (KBr): *v* = 3448 (ss, b), 2934 (w, b), 1709 (ss), 1627 (s), 1353 (m), 1094 (ss, b), 696 (w) cm⁻¹
ESI-MS (4.0 kV, MeOH): m/z (%) 816.9 ([M+Na]⁺, 100)

| C₂₇H₄₁N₃O₁₂Pt [794.71] | | | | |
|---|---|---|---|---|
| Elementaranalyse: | berechnet: gefunden: | C: 40.81 % C: 39.88 % | H: 5.20 % H: 5.16 % | N: 5.29 % N: 5.08 % |

### Beispiel 10

### Wirksamkeit von DACH-Pt-CB-1-Mal in vivo

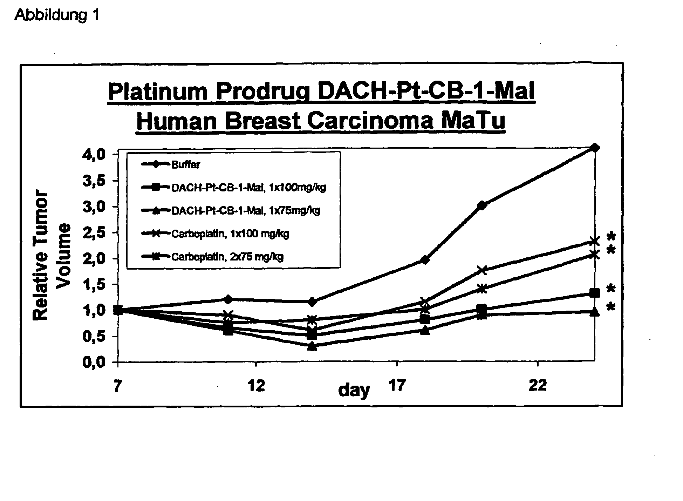

Die in Abbildung 1 aufgeführten biologischen Daten verdeutlichen eine erhöhte in-vivo-Wirksamkeit von DACH-Pt-CB-1-Mal im Vergleich zu Carboplatin.

**Tiere:** Nacktmäuse **Tumormodell:** MaTu (Mammakarzinom) **Therapie:** DACH-Pt-CB-1-Mal (75 und 100 mg/kg) sowie Carboplatin (100 mg/kg) einmalig an Tag 7, Carboplatin (75 mg/kg) jeweils an den Tagen 7 und 14; i.v. (Carboplatin und Derivate in jeweils 0.15 0.3 mL Glucose-Phosphat-Puffer pH 6.5).

## Patentansprüche

1. Platinkomplex der allgemeinen Formel I: 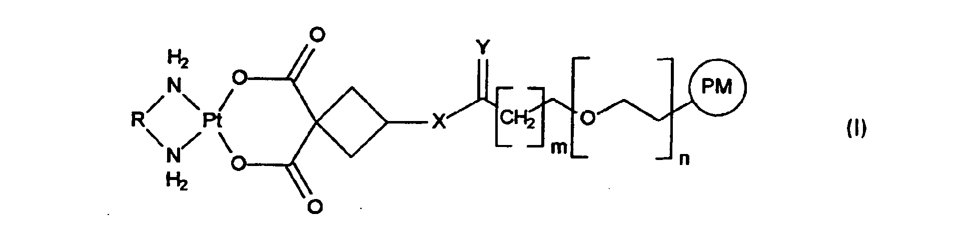 worin
R = 2 H, 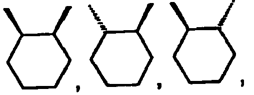 -(CH₂)ᵢ- (i = 2 oder 3);
X = O oder NH;
Y = O S oder 2 H;
m 0 bis 5;
n = 0 bis 6;
PM eine Proteinbindende Gruppe bedeutet.

2. Platinkomplex gemäß Anspruch 1,
**dadurch gekennzeichnet,**
**dass** PM eine Maleinimidgruppe, eine 2-Dithiopyridylgruppe, eine Halogenacetamidgruppe, eine Halogenacetatgruppe, eine Disulfidgruppe, eine Acrylsäureestergruppe, eine Monoalkylmaleinsäureestergruppe, eine Monoalkylmaleaminsäureamidgruppe, eine N-Hydroxysuccinimidylestergruppe, eine Isothiocyanatgruppe oder eine Aziridingruppe ist, die gegebenenfalls substituiert sein können.

3. Platinkomplex nach Anspruch 2,
**dadurch gekennzeichnet,**
**dass** PM eine Maleinimidgruppe ist, die gegebenenfalls substituiert sein kann.

4. Platinkomplex gemäß Anspruch 3,
**dadurch gekennzeichnet,**
**dass** m < 2 und n = 1 bis 4 ist.

5. Platinkomplex nach Anspruch 4,
**dadurch gekennzeichnet,**
**dass** X = O und Y = O ist.

6. Verfahren zur Herstellung von Platinkomplexen nach einem der vorhergehenden Ansprüche,
**dadurch gekennzeichnet,**
**dass** ein Cyclobutan-1,1-dicarbonsäure-Derivat der allgemeinen Formel IV 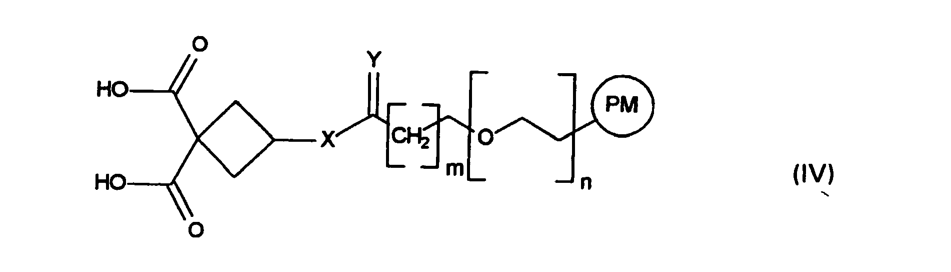 in der
X = O oder NH
Y = O, S oder 2 H
m = 0 bis 5
n = 0 bis 6
und PM eine Proteinbindende Gruppe bedeuten, mit einem Platinkomplex der allgemeinen Formel V 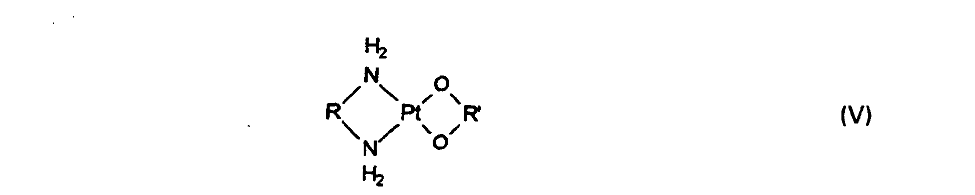 in der
R = 2 H, 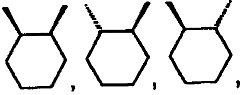 -(CH₂)ᵢ- (i = 2 oder 3)
R' = 2 NO₂, SO₂ oder CO bedeuten, umgesetzt wird.

7. Verfahren nach Anspruch 6,
**dadurch gekennzeichnet,**
**dass** das Cyclobutan-1,1-dicarbonsäure-Derivat der allgemeinen Formel II durch Umsetzung eines 4-Methoxybenzyl-geschützen Cyclobutan-1,1-dicarbonsäure-Derivats der allgemeinen Formel VII 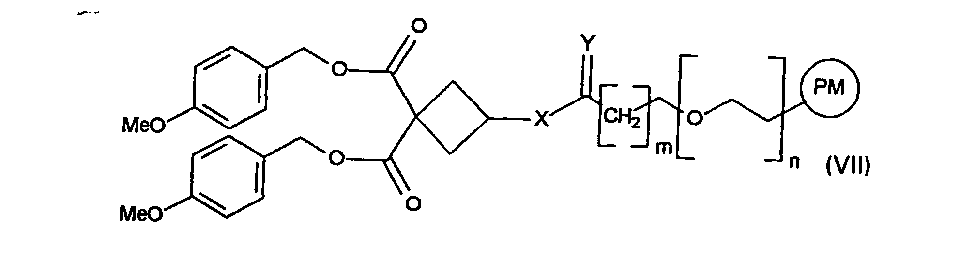 in der
X = O oder NH
Y = O, S oder 2 H
m = 0 bis 5
n = 0 bis 6
und PM eine Proteinbindende Gruppe bedeuten, mit Trifluoressigsäure und Anisol erhalten wird.

8. Verfahren nach Anspruch 7,
**dadurch gekennzeichnet,**
**dass** das Cyclobutan-1,1-dicarbonsäure-Derivat der allgemeinen Formel VII durch Umsetzung von Bis(4-methoxybenzyl)-3-hydroxycyclobutan-1,1-dicarboxylat mit einem heterobifunktionellen Crosslinker der allgemeinen Formel VI 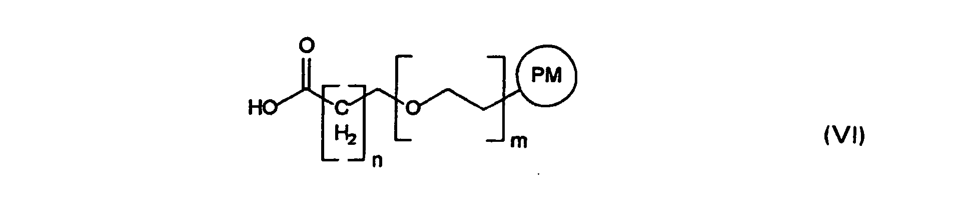 in der
n = 0, 1
m = 1 bis 6
und PM eine Proteinbindende Gruppe bedeuten, in Gegenwart von Carbonsäure-Aktivierungsreagenzien erhalten wird.

9. Verfahren nach Anspruch 8,
**dadurch gekennzeichnet,**
**dass** als Carbonsäure-Aktivierungsreagenzien *N,N'*-Dicyclohexylcarbodiimid, *N,N'*-Diisopropylcarbodiimid oder (Benzotriazol-1-yloxy)tris(dimethylamino)phosphonium-Hexafluoro-phosphat, am meisten bevorzugt 2-Chlor-1-methylpyridiniumiodid verwendet werden.

10. Verfahren gemäß Anspruch 8 oder 9,
**dadurch gekennzeichnet,**
**dass** man Bis(4-methoxybenzyl)-3-hydroxycyclobutan-1,1-dicarboxylat mit einer Maleinimidocarbonsäure der allgemeinen Formel Vla 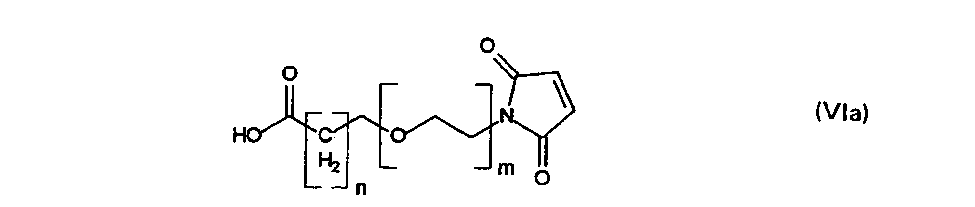 in der
n = 0, 1
m = 1 bis 6
bedeuten, unter Einsatz von 2-Chlor-1-methylpyridiniumiodid umsetzt.

11. Verfahren nach Anspruch 8,
**dadurch gekennzeichnet,**
**dass** Bis(4-methoxybenzyl)-3-hydroxycyclobutan-1,1-dicarboxylat durch Umsetzung von Bis(4-methoxybenzyl)-3-tert.-butyldimethylsiloxycyclobutan-1,1-di-carboxylat mit Tetrabutylammoniumfluorid erhalten wird.

12. Verfahren nach Anspruch 11,
**dadurch gekennzeichnet,**
**dass** Bis(4-methoxybenzyl)-3-*tert*.-butyldimethylsiloxycyclo-butan-1,1-di-carboxylat durch Umsetzung von Bis(4-methoxybenzyl)malonat mit 1,3-Dibrom-2-tert.-butyldimethyl-siloxypropan erhalten wird.

13. Arzneimittel enthaltend als Wirkstoff einen Platinkomplex gemäß einem der Ansprüche 1 bis 5, gegebenenfalls zusammen mit üblichen Hilfsstoffen und/oder pharmazeutischen Lösungsmitteln.

14. Verwendung eines Platinkomplexes nach einem der Ansprüche 1 bis 5 zur Herstellung eines Arzneimittels zur Behandlung von Krebskrankheiten.

15. Verfahren zur Herstellung eines Arzneimittels für die Behandlung von Krebskrankheiten,
**dadurch gekennzeichnet,**
**dass** man eine Verbindung gemäß einem der Ansprüche 1 bis 5 in eine therapeutisch annehmbare Lösung überführt.

## Claims

1. Platinum complex of the general formula I: 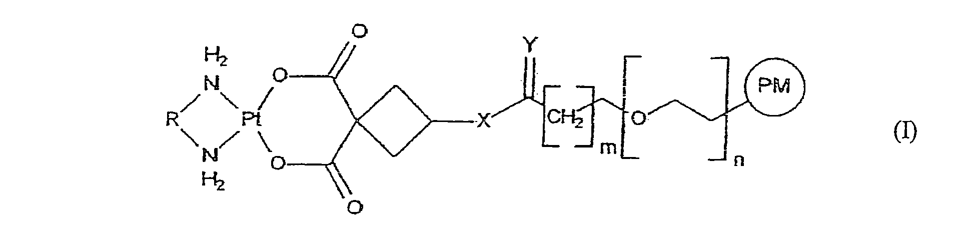 in which
R=2H, 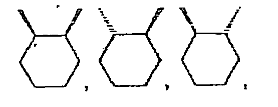 -(CH₂)ᵢ- (i = 2 or 3);
X = O or NH;
Y = O, S or 2 H;
in = 0 to 5;
n = 0 to 6;
PM denotes a protein-binding group.

2. Platinum complex as claimed in claim 1,
**characterized in that**
PM is a maleinimide group, a 2-dithiopyridyl group, a halogen acetamide group, a halogen acetate group, a disulfide group, an acrylic acid ester group, a monoalkylmaleic acid ester group, a monoalkylmaleaminic acid amide group, an N-hydroxysuccinimidyl ester group, an isothiocyanate group or an aziridine group which can be optionally substituted.

3. Platinum complex as claimed in claim 2,
**characterized in that**
PM is a maleinimide group which can be optionally substituted.

4. Platinum complex as claimed in claim 3,
**characterized in that**
m < 2 and n = 1 to 4.

5. Platinum complex as claimed in claim 4,
**characterized in that**
X=O and Y=O.

6. Process for producing platinum complexes-as claimed in one of the previous claims,
**characterized in that**
a cyclobutane-1,1-dicarboxylic acid derivative of the general formula IV 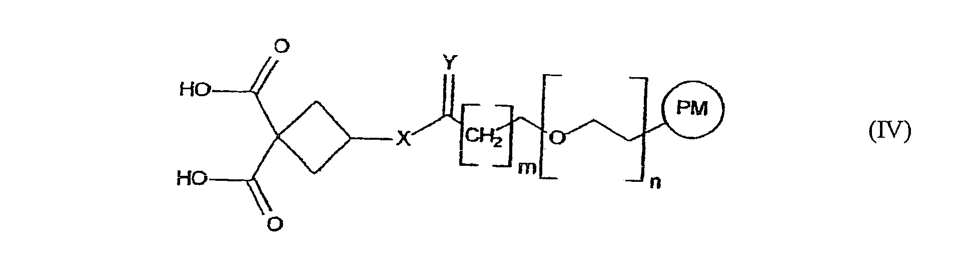 in which
X = O or NH
Y = O, S or 2 H
m = 0 to 5
n = 0 to 6
and PM denotes a protein-binding group, is reacted with a platinum complex of the general formula V 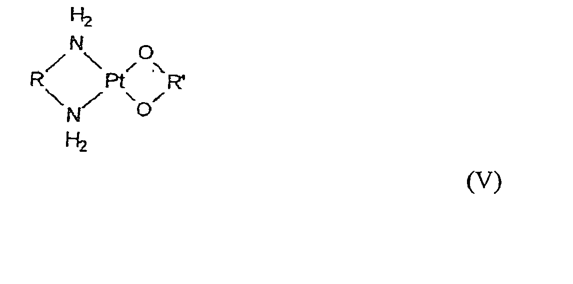 in which
R=2H, 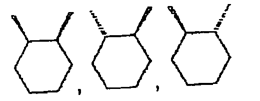 -(CH₂)ᵢ- (i = 2 or 3)
R' = 2 NO₂, SO₂ or CO.

7. Process as claimed in claim 6,
**characterized in that**
the cyclobutane-1,1-dicarboxylic acid derivative of the general formula II is obtained by reacting a 4-methoxybenzyl-protected cyclobutane-1-, 1-dicarboxylic acid derivative of the general formula VII 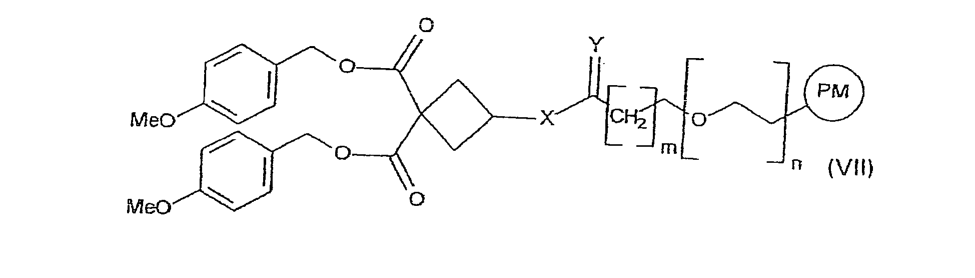 in which
X = O or NH
Y = O, S or 2H
m = 0 to 5
n = 0 to 6
and PM denotes a protein-binding group, with trifluoroacetic acid and anisole.

8. Process as claimed in claim 7,
**characterized in that**
the cyclobutane-1,1-dicarboxylic acid derivative of the general formula VII is obtained by reacting bis(4-methoxybenzyl)-3-hydroxycyclobutane-1,1-dicarboxylate with a heterobifunctional cross-linker of the general formula VI 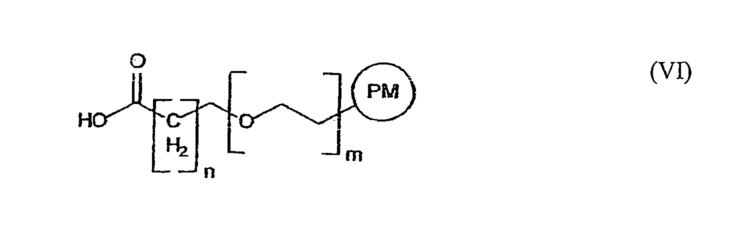 in which
n = 0,1
m= 1 to 6
and PM denotes a protein-binding group, in the presence of carboxylic acid activation reagents.

9. Process as claimed in claim 8,
**characterized in that**
*N,N*'-dicyclohexylcarbodiimide, *N,N'*-diisopropylcarbodiimide or (benzotriazole-1-yloxy)tris(dimethylamino)phosphonium hexafluorophosphate and most preferably 2-chloro-1-methylpyridinium iodide are used as carboxylic acid activation reagents.

10. Process as claimed in claim 8 or 9,
**characterized in that**
bis(4-methoxybenzyl)-3-hydroxycyclobutane-1,1-dicarboxylate is reacted with a maleinimidocarboxylic acid of the general formula VIa 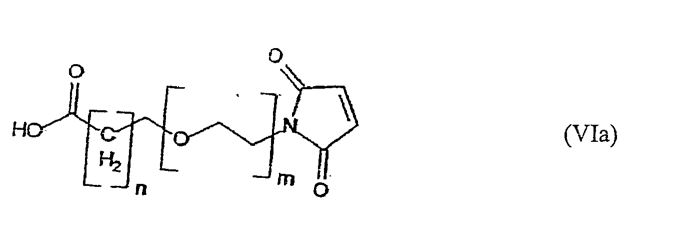 in which
n = 0,1
m = 1 to 6
using 2-chloro-1-methylpyridinium iodide.

11. Process as claimed in claim 8,
**characterized in that**
bis(4-methoxybenzyl)-3-hydroxycyclobutane-1,1-dicarboxylate is obtained by reacting bis(4-methoxybenzyl)-*3-tert.*-butyldimethylsiloxycyclobutane-1,1-dicarboxylate with tetrabutylammonium fluoride.

12. Process as claimed in claim 11,
**characterized in that**
bis(4-methoxybenzyl)-3-*tert*.-butyldimethylsiloxycyclobutane-1,1-dicarboxylate is obtained by reacting bis(4-methoxybenzyl)malonate with 1,3-dibromo-2-*tert*. -butyldimethylsiloxypropane.

13. Pharmaceutical preparation containing a platinum complex according to any one of the claims 1 to 5 as an active ingredient, optionally together with common auxiliary substances and/or pharmaceutical solvents.

14. Use of a platinum complex as claimed in any one of the claims 1 to 5 for the manufacture of a pharmaceutical preparation for the treatment of cancer diseases.

15. Process for producing a pharmaceutical preparation for treating cancer diseases,
**characterized in that**
a compound as claimed in any one of the claims 1 to 5 is transferred into a therapeutically acceptable solution.

## Revendications

1. Complexe de platine de formule générale I: 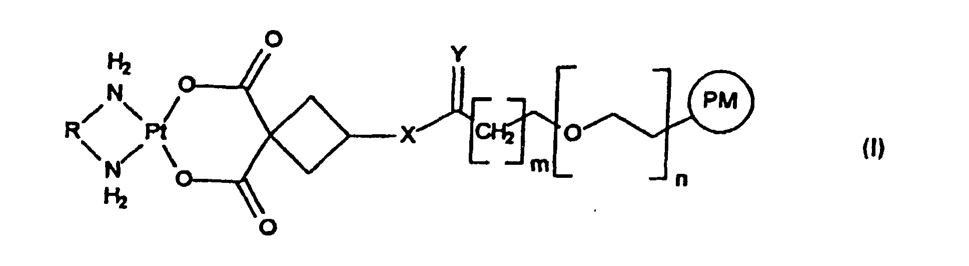 dans laquelle
R=2H, 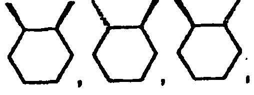 -(CH₂)ᵢ- (i = 2 ou 3);
X = O ou NH;
Y = O, S ou 2 H;
m = 0 à 5;
n = 0 à 6;
PM représente un groupe de liaison à une protéine.

2. Complexe de platine selon la revendication 1, **caractérisé en ce que** PM est un groupe maléimide, un groupe 2-dithiopyridyle, un groupe halogénoacétamide, un groupe halogénoacétate, un groupe disulfure, un groupe ester d'acide acrylique, un groupe ester monoalkylique d'acide maléique, un groupe amide d'acide monoalkylmaléamique, un groupe ester de N-hydroxysuccinimidyle, un groupe isothiocyanate ou un groupe aziridine, qui peuvent éventuellement être substitués.

3. Complexe de platine selon la revendication 2, **caractérisé en ce que** PM est un groupe maléimide qui peut éventuellement être substitué.

4. Complexe de platine selon la revendication 3, **caractérisé en ce que** m < 2 et n = 1 à 4.

5. Complexe de platine selon la revendication 4, **caractérisé en ce que** X = O et Y = O.

6. Procédé de préparation de complexes de platine selon l'une des revendications précédentes, **caractérisé en ce que** l'on fait réagir un dérivé d'acide cyclobutane-1,1-dicarboxylique de formule générale IV 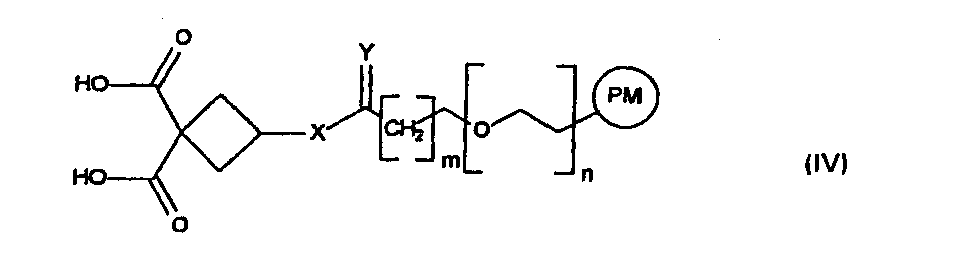 dans laquelle
X = O ou NH;
Y = O, S ou 2 H;
m = 0 à 5;
n = 0 à 6;
et PM représente un groupe de liaison à une protéine, avec un complexe de platine de formule générale V 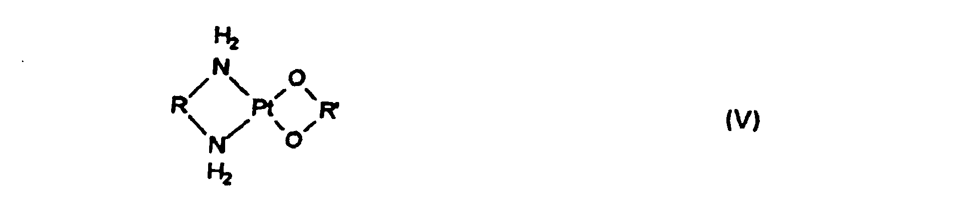 dans laquelle
R = 2H, 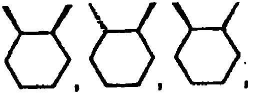 -(CH₂)ᵢ- (i = 2 ou 3);
R' = 2 NO₂, SO₂ ou CO.

7. Procédé selon la revendication 6, **caractérisé en ce que** le dérivé d'acide cyclobutane-1,1-dicarboxylique de formule générale II est obtenu par réaction d'un dérivé d'acide cyclobutane-1,1-dicarboxylique protégé par des groupes 4-méthoxybenzyle de formule générale VII 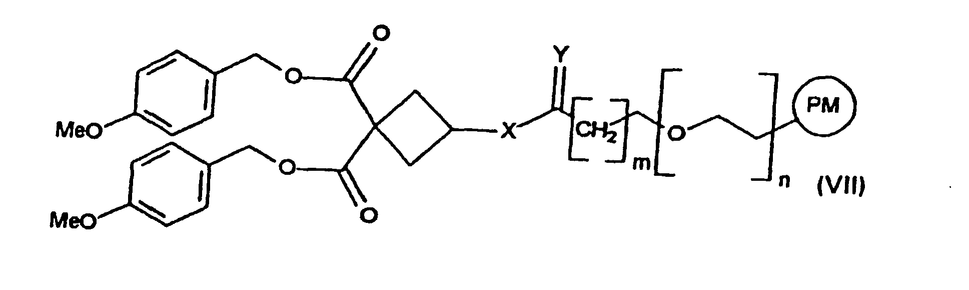 dans laquelle
X = O ou NH;
Y = O, S ou 2 H;
m = 0 à 5;
n = 0 à 6;
et PM représente un groupe de liaison à une protéine, avec de l'acide trifluoroacétique et de l'anisole.

8. Procédé selon la revendication 7, **caractérisé en ce que** le dérivé d'acide cyclobutane-1,1-dicarboxylique de formule générale VII est obtenu par réaction de 3-hydroxycyclobutane-1,1-dicarboxylate de bis(4-méthoxybenzyle) avec un agent réticulant hétérobifonctionnel de formule générale VI 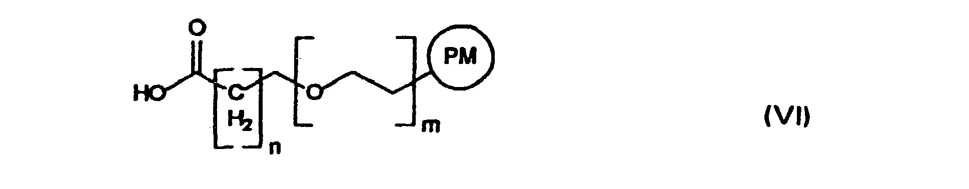 dans laquelle
n = 0, 1
m = 1 à 6
et PM représente un groupe de liaison à une protéine, en présence de réactifs d'activation d'acides carboxyliques.

9. Procédé selon la revendication 8, **caractérisé en ce que** l'on utilise comme réactifs d'activation d'acides carboxyliques le N,N'-dicyclohexylcarbodiimide, le N,N'-diisopropylcarbodiimide ou l'hexafluorophosphate de (benzotriazol-1-yloxy)tris(diméthylamino)phosphonium, de préférence l'iodure de 2-chloro-1-méthylpyridinium.

10. Procédé selon la revendication 8 ou 9, **caractérisé en ce que** l'on fait réagir le 3-hydroxycyclobutane-1,1-dicarboxylate de bis(4-méthoxybenzyle) avec un acide maléimidocarboxylique de formule générale VIa 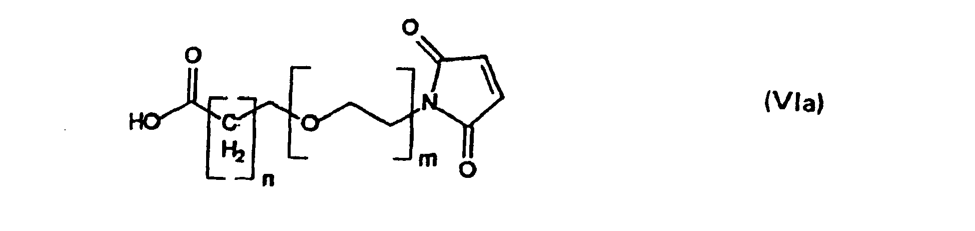 dans laquelle
n = 0, 1
m = 1 à 6
en utilisant de l'iodure de 2-chloro-1-méthylpyridinium.

11. Procédé selon la revendication 8, **caractérisé en ce que** le 3-hydroxycyclobutane-1,1-dicarboxylate de bis(4-méthoxybenzyle) est obtenu par réaction de 3-tert-butyldiméthylsiloxycyclobutane-1,1-dicarboxylate de bis(4-méthoxybenzyle) avec du fluorure de tétrabutylammonium.

12. Procédé selon la revendication 11, **caractérisé en ce que** le 3-tert-butyldiméthylsiloxycyclobutane-1,1-dicarboxylate de bis(4-méthoxybenzyle) est obtenu par réaction de malonate de bis(4-méthoxybenzyle) avec du 1,3-dibromo-2-tert-butyldiméthylsiloxypropane.

13. Médicament contenant, comme substance active, un complexe de platine selon l'une des revendications 1 à 5, éventuellement avec des agents auxiliaires classiques et/ou des solvants pharmaceutiques.

14. Utilisation d'un complexe de platine selon l'une des revendications 1 à 5 pour la préparation d'un médicament destiné au traitement de maladies cancéreuses.

15. Procédé de préparation d'un médicament destiné au traitement de maladies cancéreuses, **caractérisé en ce que** l'on fait passer un composé selon l'une des revendications 1 à 5 en une solution thérapeutiquement acceptable.
